(19) Europäisches Patentamt
European Patent Office
Office européen des brevets

(11) Publication number: **0 125 033**
**B1**

(12) # EUROPEAN PATENT SPECIFICATION

(45) Date of publication of patent specification: **26.04.89**

(21) Application number: **84302423.3**

(22) Date of filing: **10.04.84**

(51) Int. Cl.⁴: **C 07 D 233/84,**
C 07 C 157/09, C 07 C 143/80,
C 07 C 147/06, C 07 C 101/42,
C 07 C 103/29, C 07 C 121/80,
C 07 C 93/04, C 07 C 93/14,
A 61 K 31/415

(54) **Dopamine-beta-hydroxylase inhibitors.**

(30) Priority: **12.04.83 US 484122**
**19.03.84 US 590665**

(43) Date of publication of application:
**14.11.84 Bulletin 84/46**

(45) Publication of the grant of the patent:
**26.04.89 Bulletin 89/17**

(84) Designated Contracting States:
**AT BE CH DE FR GB IT LI LU NL SE**

(56) References cited:
**DE-A-2 245 467**
**DE-A-2 843 016**
**DE-C- 825 253**
**GB-A-1 155 580**
**GB-A-1 291 524**
**GB-A-2 062 626**
**US-A-3 767 816**
**US-A-3 915 980**

(73) Proprietor: **SMITHKLINE BECKMAN CORPORATION**
**P.O. Box 7929 1 Franklin Plaza**
**Philadelphia Pennsylvania 19101 (US)**

(72) Inventor: **Frazee, James Simpson**
**310B Sutton Towers**
**Collingswood, NJ 08108 (US)**
Inventor: **Kaiser, Carl**
**1105 Sylvan Drive**
**Haddon Heights, NJ 08035 (US)**
Inventor: **Kruse, Lawrence Ivan**
**646 Clinton Avenue**
**Haddonfield, NJ 08033 (US)**

(74) Representative: **Waters, David Martin, Dr. et al**
**Smith Kline & French Laboratories Ltd. Patent Department Mundells**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 125 033 B1

## Description

Field of the Invention

This invention relates to inhibitors of dopamine-ß-hydroxylase.

Background of the Invention

In the catecholamine biosynthetic pathway, tyrosine is converted in three steps to norepinephrine (NE). Intermediates are dihydroxyphenylalanine (DOPA) and dopamine (DA). The latter is hydroxylated to norephinephrine by dopamine-ß-hydroxylase (DBH) in the presence of oxygen and ascorbic acid.

Inhibition of catecholamine activity has been found to decrease hypertension. See, for example, Matta et al. *Clin. Pharm. Ther. 14,* 541 (1973), and Teresawa et al. *Japan Circ. J. 35,* 339 (1971). Weinshilboum, *Mayo Clin. Proc. 55,* 39 (1980), reviews compounds which inhibit catecholamine activity by interfering with adrenergic receptors. Alternatively, the catecholamine biosynthetic pathway can be suppressed at any of the three steps, resulting in decreased levels of NE. In addition to decreasing hypertension, inhibitors of NE synthesis are active as diuretics, natriuretics, cardiotonics and vasodilators. Inhibition of DBH activity can have the added advantage of increasing levels of DA, which as reported by Ehrreich et al., "New Antihypertensive Drugs," Spectrum Publishing, 1976, pp. 409—432, has been found to have selective vasodilator activity at certain concentrations.

DBH inhibitors have also been shown to reduce or prevent formation of gastric ulcers in rats by Hidaka et al., "Catecholamine and Stress." edit. by Usdin et al, Permagon Press, Oxford, 1976, pp. 159—165 and by Osumi et al., *Japan. J. Pharmacol. 23,* 904 (1973).

Although there are many known inhibitors of DBH, none of these agents has found clinical application because of non-specific, often toxic, properties they possess. Fusaric acid, for example, has been found to be hepatotoxic. See, for example, Teresawa et al., *Japan. Cir. J. 35,* 339 (1971) and reference cited therein. Presumably, the picolinic acid structure interacts with a number of metalloproteins and enzymes in non-specific fashion to produce observed side effects.

In U.K. specification 1,155,580 are disclosed compounds having the formula:

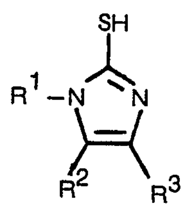

wherein $R^2$ and $R^3$ can be H and $R^1$ can be substituted phenyl. The compounds are said to have analgesic, anti-inflammatory and antipyretic properties. Gebert et al., U.S. Patent 3,915,980, disclose such compounds wherein $R^1$ can be phenyl or phen($C_{1-3}$) alkyl, as intermediates to imidazolyl-2-thioalkanoic acid esters.

Iverson, *Acta Chem. Scand. 21,* 279 (1967) reports a compound having the formula:

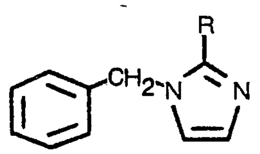

wherein R can be $—CO_2H$ or $—CH_2NHC_6H_5$, but does not report a pharmaceutical use for the compound.

Summary of the Invention

The invention resides in the discovery that DBH can be inhibited by a compound having a mercaptoimidazole moiety and a phenethylamine analogue moiety. More particularly, the invention is selected novel compounds having the formula:

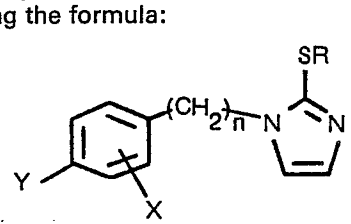

wherein

X is —H, —OH, halogen, $C_{1-4}$ alkyl, —CN, $—NO_2$, $—SO_2NH_2$, $—CO_2H$, $—CONH_2$, —CHO, $—CH_2OH$, $—CF_3$, $—OCH_3$, $—SO_2C_{1-4}$ alkyl, $—SO_2C_{1-4}$ fluoroalkyl, $—CO_2C_{1-4}$ alkyl or any synthetically accessible combination thereof up to four substituents;

Y is —H, —OH, halogen, $C_{1-4}$ alkyl, —CN, $—NO_2$, $—SO_2NH_2$, $—CO_2H$, $—CONH_2$, —CHO, $—CH_2OH$, $CF_3$, $—SO_2C_{1-4}$ alkyl, $—SO_2C_{1-4}$ fluoroalkyl, or $—CO_2C_{1-4}$ alkyl;

R is —H or $C_{1-4}$ alkyl; and n is 0—4, or a hydrate or, when R is $C_{1-4}$ alkyl, a pharmaceutically acceptable acid addition salt thereof, provided that when n is 0, Y is —OH and when n is 1—3, at least one of Y and X is not —H.

In preferred compounds of the invention, Y is —OH; R is —H; n is 1 or 3; and X is —H, —OH or halogen (in particular, 3,5-dichloro, 3,5-difluoro, 3-chloro, or 3-fluoro) or Y is —H; R is —H; n is 1 or 3 and X is halogen (in particular 3,5-dichloro, 3,5-difluoro, 3-chloro, or 3-fluoro). In the most preferred compound of the invention, Y is —H, X is 3,5-difluoro, R is —H and n is 1.

The invention is also compounds of the following formula for use in inhibiting DBH activity in mammals:

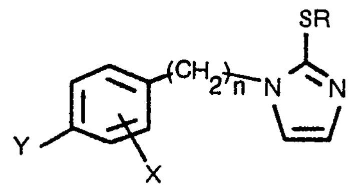

wherein

X is —H, —OH, halogen, $C_{1-4}$ alkyl, —CN, $—NO_2$, $—SO_2NH_2$, $—CO_2H$, $—COHN_2$, —CHO, $—CH_2OH$, $—CF_3$, $—OCH_3$, $—SO_2C_{1-4}$ alkyl. $—SO_2C_{1-4}$ fluoroalkyl, $—CO_2C_{1-4}$ alkyl or any synthetically accessible combination thereof of four substituents;

Y is —H, —OH, halogen, $C_{1-4}$ alkyl, —CN, $—NO_2$, $—SO_2NH_2$, $—CO_2H$, $—CONH_2$, —CHO, $—CH_2OH$, $—CF_3$, $—SO_2C_{1-4}$ alkyl. $—SO_2C_{1-4}$ fluoroalkyl, or $—CO_{21-4}$ alkyl;

R is —H or $C_{1-4}$ alkyl; and n is 0—4, or a hydrate or when R is $C_{1-4}$ alkyl, a pharmaceutically acceptable acid addition salt thereof.

Compounds found to be especially potent and therefore preferred for use as dopamine-ß-hydroxylase inhibitors are those in which Y is —OH; R is —H; n is 1 or 3; and X is —H, —OH or halogen (in particular, 3,5-dichloro, 3,5-difluoro, 3-chloro or 3-fluoro) or Y is —H; R is —H; n is 1 or 3 and X is halogen (in particular, 3,5-dichloro, 3,5-difluoro, 3-chloro, or 3-fluoro). In the most preferred method of the invention, Y is —H; X is 3,5-difluoro, R is —H and n is 1.

It is intended that the above formulae include the tautomer of the compounds wherein R is —H, that is, the compounds having the above formulae wherein the imidazole moiety has the formula:

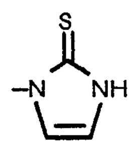

The above formulae also include hydrates of the compounds and pharmaceutically acceptable acid addition salts of the compounds wherein R is $C_{1-4}$ alkyl. The invention also includes pharmaceutical compositions comprising the compounds having the above formulae, provided that when n is 0, Y is —OH, and pharmaceutical carriers.

The intermediates to the compounds of the invention, have the formula:

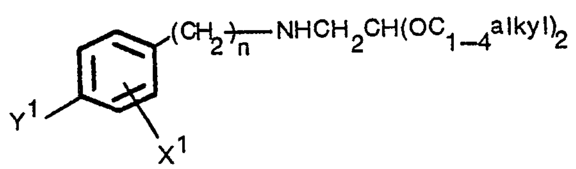

II

wherein $Y^1$ and $X^1$ are the same as Y and X but are not —OH and n is 0—4 and

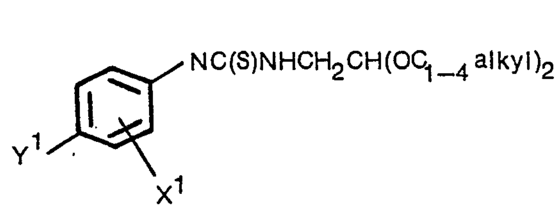

IIA

wherein $X^1$ is the same as X but is not —OH, $Y^1$ is $—OCH_3$ and n is 0.

The invention is also a process for preparing the compound of the invention which comprises contacting and reacting compound II, above, with acidic thiocyanate and such process which comprises

contacting and reacting compound II A, above, with an acid to cyclize the compound. In both processes, when $Y^1$ and/or $X^1$ are —$OCH_3$, Y and/or X are optionally deprotected to prepare the compound wherein Y and/or X are —OH.

Detailed Description of the Invention

The compounds of the present invention contain weak metal-chelating functional groups derived from N-methyl-2-mercaptoimidazole which is known to be a weak DBH inhibitor. The compounds of the invention also contain phenyl moieties as do phenethylamine analogue inhibitors such as benzyloxyamine, benzylhydrazine, tryptamine and serotonin.

The compounds of the invention and the compounds used for inhibiting dopamine-ß-hydroxylase activity can be prepared from corresponding starting benzyl or phenyl compounds such as benzaldehydes, which are known and described in published references or are readily accessible, by known techniques such as illustrated in Scheme I, below, wherein $X^1$ and $Y^1$ are X and Y, respectively, except that when Y is —OH, $Y^1$ is —$OCH_3$ and when X is —OH, $X^1$ is —$OCH_3$. As illustrated, n is one, although n can be 0—4. Scheme I illustrates reductive amination of benzaldehydes (I) with an aminoacetaldehyde acetal followed by reduction by, for example, catalytic hydrogenation or treatment with a reducing agent such as $NaBH_4$, $LiAlH_4$ or $AlH_3$, to provide intermediate substituted benzylamines (II). Upon reaction with acidic thiocyanate, the intermediates (II) yield mercaptoimidazole products (III). The mercaptoimidazole products can be prepared from other than benzaldehydes, as illustrated in Examples 1 and 4 below.

SCHEME I

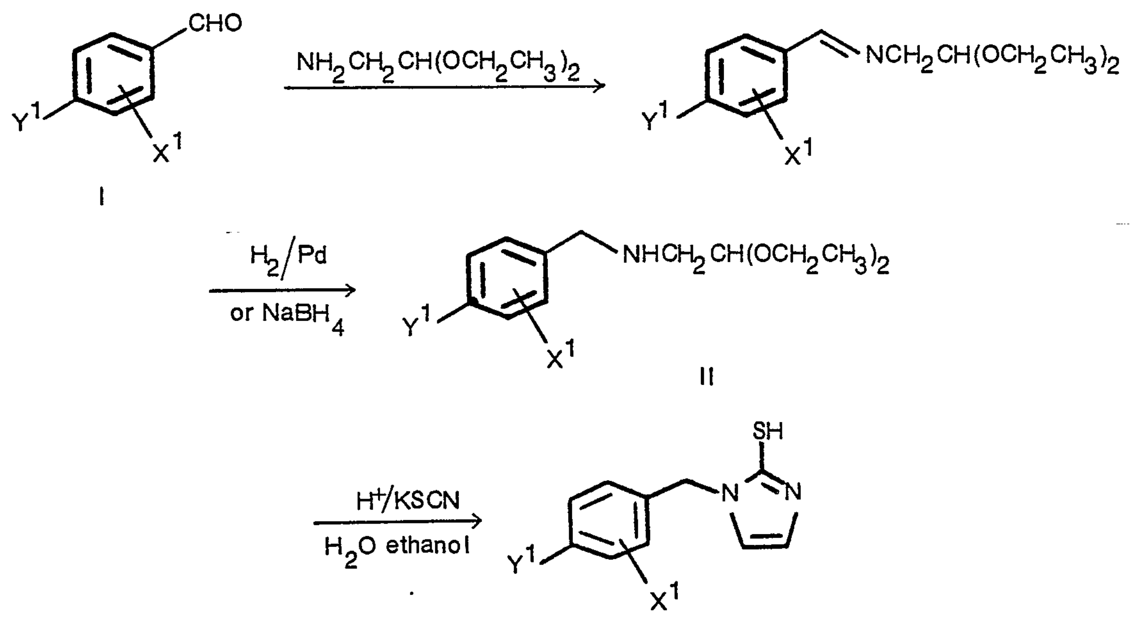


I

II

III

The 1-phenyl substituted 2-mercaptoimidazoles (n is 0) are preferably prepared by reaction of an appropriately substituted phenyl isothiocyanate with an aminoacetaldehyde acetal followed by strong acid catalyzed cyclization, as illustrated in Example 1, below.

The compounds wherein n is 2, 3 or 4 are preferably prepared as illustrated in Example 4 and in Examples 23 and 24, below. Coupling of substituted phenylalkanoic acids as the acid halides, preferably chlorides, with aminoacetaldehyde acetals and subsequent reduction provides such intermediate substituted phenyl alkylamines.

$Y^1$ in Scheme I is the same as Y except that when Y is —OH, $Y^1$ is —$OCH_3$, deprotection of the 4-alkoxy group with, for example, $BBr_3$ or HBr, or nucleophilic aromatic substitution with dilute hydroxide, provides the phenol (Y is —OH). $X^1$ may be one or more substituents at the 2-, 3-, 5- or 6- positions, provided the combination of substituents is accessible, that is does not result in significantly instability due to steric hindrance. When $X^1$ is —$OCH_3$, it can be deprotected as decribed above for $Y^1$.

The compounds in which R is $C_{1-4}$ alkyl are preferably prepared by allowing the deprotection with for example, $BBr_3$, in an alkanol to proceed to formation of an alkyl bromide which alkylates the mercapto group as illustrated in Example 6, below. Alternatively, a solution or suspension of an appropriately substituted mercaptoimidazole in an inert solvent, for example, methanol, tetrahydrofuran and aqueous dimethylformamide, can be reacted with an alkylating agent, for example, alkyl iodide, bromide or tosylate. Methyl iodide is preferred in this alternative procedure.

The pharmaceutically acceptable acid addition salts of the compounds wherein R is $C_{1-4}$ alkyl are formed with strong or moderately strong organic or inorganic acids by methods known to the art. For example, the base is reacted with an inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of the salts which are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts.

The compounds of the invention, because they can be used to inhibit DBH activity, have therapeutic value as diuretic, natriuretic, carditonic, antihypertensive and vasodilator agents, as well as antiulcerogenic and antiparkinson disease agents.

Compounds of the invention and other compounds useful in the method of the invention were screened for *in vitro* DBH inhibition by a standard procedure for assaying converson of tyramine to octopamine in the presence DBH. Octopamine was assayed following sodium periodiate oxidation to p-hydroxybenzaldehyde by measuring spectrophotometric absorbance at 330 nm. Results are given in Table I, below. Inhibition is given in molar concentration of compound at which DBH activity was halved ($IC_{50}$). Melting points (mp) are given in °C. By this procedure fusaric acid was found to have an $IC_{59}$ of about $8 \times 10^{-7}$.

TABLE I

| n | X | Y | R | mp | $IC_{50}$ |
|---|---|---|---|---|---|
| 1 | 3—Br | OH | H | 181 | $1.2 \times 10^{-5}$ |
| 1 | 3—F | OH | H | 172 | $1.4 \times 10^{-6}$ |
| 1 | 3—OH | H | H | 167 | $1.5 \times 10^{-4}$ |
| 1 | 2—OH | H | H | 158 | $5.8 \times 10^{-4}$ |
| 1 | 3—$CH_3$ | OH | H | 214—216 | $4.8 \times 10^{-5}$ |
| 1 | 2—$OCH_3$ | H | H | 159 | $10^{-4}$ (68% inhibition) |
| 1 | 3—$OCH_3$ | H | H | 181—121 | $10^{-4}$ (72% inhibition) |
| 4 | H | OH | H | 132—134 | $10^{-4}$ (12% inhibition) |
| 3 | H | OH | $CH_3$ | 140—142 | $7.9 \times 10^{-5}$ |
| 1 | 3,5—$C1_2$ | OH | H | 220—222 (dec) | $7.4 \times 10^{-7}$ |
| 1 | 3—$NO_2$ | OH | H | 224—227 | $2.0 \times 10^{-5}$ |
| 1 | 2,6—$Cl_2$ | OH | H | >235 (dec) | $7.5 \times 10^{-5}$ |
| 1 | 3,5—$F_2$ | OH | H | 213—215 | $7.4 \times 10^{-8}$ |
| 1 | 3—$CF_3$ | OH | H | 220 (dec) | $1.2 \times 10^{-4}$ |
| 1 | 2,3,5,6—$F_4$ | OH | H | 203—5 | $6.2 \times 10^{-5}$ |
| 1 | H | H | H | 144—5 | $1.1 \times 10^{-5}$ |
| 2 | H | OH | H | 181—184 | $1.8 \times 10^{-5}$ |
| 0 | H | H | H | 180—182 | $1.0 \times 10^{-4}$ |
| 1 | H | OH | H | 188—190 | $2.3 \times 10^{-6}$ |
| 3 | H | OH | H | 183—185 | $2.0 \times 10^{-6}$ |
| 1 | 3—OH | OH | H | 209—212 | $4.0 \times 10^{-6}$ |
| 0 | H | OH | H | 260—264 | $3.2 \times 10^{-4}$ |
| 1 | 3—Cl | OH | H | 186—190 | $2 \times 10^{-6}$ |
| 1 | 2,6—$Cl_2$ | H | H | 242—243 | $10^{-4}$ (7% inhibition) |
| 1 | 2—Cl | H | H | 206—207 | $10^{-4}$ (14% inhibition) |
| 1 | 2,5—$Cl_3$ | H | H | 265 (dec) | $9.7 \times 10^{-5}$ |
| 1 | 4—Cl | H | | 187—189 | $9.6 \times 10^{-5}$ |
| 1 | 2,3—$Cl_2$ | H | H | 195—197 | $5.2 \times 10^{-5}$ |
| 1 | H | F | H | 167—169 | $4.7 \times 10^{-5}$ |
| 1 | 3,4—$Cl_2$ | H | H | 178—181 | $2.8 \times 10^{-5}$ |
| 1 | 2,4—$Cl_2$ | H | H | 185—8187 | $1.7 \times 10^{-5}$ |
| 1 | 3—Cl | H | H | 129—131 | $1.2 \times 10^{-5}$ |
| 1 | 3,5—$Cl_2$ | H | H | 209—211 | $2.4 \times 10^{-6}$ |
| 1 | 2, 4, 6—$Cl_3$ | H | H | 240—244 | $1.0 \times 10^{-4}$ |
| 1 | 3—F | H | H | 113—114 | $5.6 \times 10^{-6}$ |
| 1 | 3,5—$F_2$ | H | H | 140—141 | $1.2 \times 10^{-6}$ |
| 3 | 3,5—$Cl_2$ | H | H | 98—99 | $2.0 \times 10^{-6}$ |
| 3 | 3,5—$F_2$ | H | H | 131—132 | $4.7 \times 10^{-6}$ |

The above results illustrate that compounds of the invention and other compounds useful in the method of the invention inhibit DBH activity.

The following procedure was used to screen compounds of the invention for activity *in vivo.* Male Okamoto-Aoki strain spontaneously hypertensive rates (SHR), 270—340 g, aged 16—20 weeks, were used for testing. The afternoon before testing, the animals were fasted and the following morning the first dose of the test compound was administered, p.o., along with a 25 ml/kg, load of normal saline. The animals were then placed in metabolism cages, three per cage, and urine was collected for three hr and subsequently analyzed for sodium, potassium, and creatinine. Indirect systolic blood pressure and heart rate were measured via a tail-cuff method and, within 24 hr of the first dose, the animals received an identical second dose of the test compound. Two hr after the second dose, the systolic blood pressure and heart rate were again determined. Drugs were administered intraperitoneally as a solution or suspension in 0.9% NaCl with 0.02% ascorbic acid. The dose volume as 5 ml.

Averaged results of the *in vivo* screens are given in Table II. In all compounds tested, R is —H and Y is —OH. Except where otherwise indicated, the dose concentration was 50 mg/kg. Averaged results with control animals are listed in parenthesis below results of treated animals.

TABLE II

| Compound | | No. of Animals | Electrolytes Excreted (μEq/rat) | | Urine Vol. (ml/rat) | $Na^+/K^+$ Ratio | Systolic Blood Pressure (mmHg) | | Heart Rate (beats/min) | |
|---|---|---|---|---|---|---|---|---|---|---|
| x | n | | $Na^+$ | $K^+$ | | | First Dose | Second Dose | First Dose | Second Dose |
| 3—Cl | 1 | 3 | 280.33 | 182.22 | 8 | 1.538 | 173 | 180 | 400 | 440 |
| | | (3) | (381.56) | (133.79) | (8) | (2.852) | (174) | (170) | (440) | (460) |
| 3—F | 1 | 3 | 162.07 | 134.81 | 3 | 1.202 | 177 | 191 | 460 | 420 |
| | | (3) | (214,16) | (198.80) | (6) | (1.077) | (188) | (189) | (460) | (440) |
| H | 3(1) | 3 | 185.81 | 160.59 | 5 | 1.157 | 181 | 185 | 440 | 440 |
| H | 3(2) | 3 | 339.19 | 231.19 | 8 | 1.467 | 187 | 180 | 380 | 440 |
| | | (3) | (390.84) | (231.19) | (8) | (1.691) | (193) | (192) | (480) | (460) |
| H | 3 | 3 | 334.84 | 104.72 | 8 | 3.197 | 181 | 163 | 460 | 460 |
| | | (3) | (183.41) | (91.11) | (4) | (2.013) | (183) | (180) | (460) | (460) |
| 3,5—$CL_2$ | 1 | 3 | 541.03 | 293.07 | 13.0 | 1.846 | 167 | 160 | 440 | 500 |
| | | (3) | (333.42) | (153.17) | (6.5) | (2.177) | (167) | (176) | (400) | (460) |
| 3—OH | 1 | 3 | 149.10 | 174,26 | 4 | 0.856 | 185 | 173 | 460 | 480 |
| | | (3) | (161.19) | (154.61) | (4) | (1.043) | (180) | (185) | (480) | (400) |
| 3—OH | 1(2) | 3 | 383.61 | 205.29 | 10 | 1.869 | 173 | 178 | 440 | 440 |
| | | (3) | (395.72) | (164.23) | (8) | (2.409) | (171) | (199) | (420) | (480) |
| | 1(1) | 3 | 369.40 | 251.79 | 11 | 1.467 | 181 | 183 | 400 | 440 |
| H | 1 | 3 | 495.00 | 229.27 | 11 | 2.159 | 193 | 182 | 480 | 480 |
| | | (3) | (344.55) | (137.84) | (9) | (2.500) | (171) | (183) | (380) | (440) |
| | 3 | 3 | 103.46 | 53.34 | 1 | 1.940 | 147 | 153 | 320 | 400 |
| 3,5—$Cl_2$* | | (3) | (146.06) | (20.11) | (2) | (7.264) | (179) | (186) | (420) | (460) |
| | 3 | 3 | 86.67 | 54.32 | 3.5 | 1.596 | 159 | 141 | 360 | 360 |
| 3,5—$Cl_2$* | | (3) | 128.60 | 77.25 | 3 | 1.665 | 172 | 182 | 400 | 480 |

(1) dose concentration = 12.5 mg/kg. *Y is —H
(2) dose concentration = 25.0 mg/kg.

It is apparent from Table II that the compounds tested have significant diuretic and/or cardiotonic activity. The compounds in which X is 3,5-dichloro showed significant natriuretic activity as well as diuretic, antihypertensive and cardiotonic activity. Compounds having diuretic activity are known to be useful as antihypertensives.

In addition experiments carried out substantially by the above procedure, the compound in which X is 3,5—$F_2$, Y is —H, R is —H and n is 1 (50 mg/kg) was found to have an especially pronounced effect on urine excretion, increasing urine volume about four-fold over controls. Heart rate was generally decreased by administration of the compound.

Various compounds of the invention, as well as various known DBH inhibitors, were tested for their effects on peripheral dopamine and norepinephrine levels substantially by the procedure of DaPrada and Zürcher, *Life Sci. 19,* 1161 (1976). Spontaneously hypertensive rats were dosed twice, the second dose being about 18 hr after the first, and were sacrificed about 2 hr after the second dose. Averaged results, expressed in micrograms of DA per gram of tissue, are given in Table III and in Table III A, which follows. In Table III, R= —H, Y= —OH and n=1. In Table III A, R= —H, Y= —H and n=1.

TABLE III

| Compound | No. of Animals | DA (ug/g) | | DA/NE Ratio | |
|---|---|---|---|---|---|
| Control ($H_2O$) | 11 | .260 | .019 | .040 | .002 |
| Fusaric Acid | 11 | .520 | .053(1) | .100 | .007(1) |
| Control | 3 | .219 | .044 | .035 | .002 |
| Hydralazine | | | | | |
| (25 mg/kg) | 3 | .417 | .026(2) | .078 | |
| (50 mg/kg) | 1 | .835 | .127 | .098 | .015(2) |
| Control | 1 | .299 | .038 | .039 | .004 |
| X=3—F (50 mg/kg) | 1 | .476 | .023(3) | .064 | .004(2) |
| Control | 1 | .261 | .046 | .041 | .007 |
| X=3—F | | | | | |
| (50 mg/kg) | 1 | .430 | .027(3) | .082 | .005(3) |
| (100 mg/kg) | 1 | .619 | .071(3) | .099 | .003(1) |
| Control | 1 | .273 | .019 | .040 | .002 |
| X=3,5—$Cl_2$ (50 mg/kg) | 1 | .241 | .031 (3) | .045 | .006 (3) |
| Control (cold-stressed) | 1 | .242 | .014 | .030 | .002 |
| X=3,6—$Cl_2$ (50 mg/kg) (cold-stressed) | 1 | .313 | .019(2) | .038 | .003(2) |
| Control | 5 | .270 | .025 | .0307 | .0019 |
| Fusari2 Acid (50 mg/kg) | 5 | .675 | .030(1) | .0871 | .0047(1) |
| X=3,5—$F_2$ (50 mg/kg) | 5 | .708 | .068(1) | .0824 | .0128(2) |

(1) $P< 0.001$ (2) $P< 0.05$ (3) $P< 0.01$

TABLE IIIA

| Compound | Number of Animals | DA (ug/g) | DA/NE Ratio |
|---|---|---|---|
| Control | 4 | .319 ± .104 | .042 ± .012 |
| Fusaric Acid | 5 | .642 ± .114 | .1240 ± .0140[1] |
| X=3—F n=1 (50 mg/kg) | 5 | 1.096 ± .080 | .1946 ± .0112[1] |
| X=3,5—$F_2$ (50 mg/kg) | 5 | 2.109 ± .123 | .4485 ± .0532[1] |
| Control | 5 | .250 ± .014 | .037 ± .003 |
| X=3,5$Cl_2$ (50 mg/kg) | 5 | .670 ± .065 | .110 ± .008[1] |
| Control | 5 | .308 ± .023 | .045 ± .004 |
| X=3,5$Cl_2$ (50 mg/kg) | 5 | .688 ± .020 | .103 ± .002[1] |

(1) P>0.001

The above results illustrate that the compounds of the invention inhibit DBH activity in mammals when administered internally in effective amounts. The compound of the invention in which X is —H, Y is —OH, R is —H and n is 3 was also tested in one rat. The results did not indicate significant inhibition of DBH activity. Nevertheless, because only a single experiment was run, because the compounds of the invention show such activity, and because the compound has a low *in vitro* $IC_{50}$ (See Table I), the compound is believed to be useful in inhibiting DBH activity in mammals.

The compounds in which Y is —H and X is halogen, especially difluoro and dichloro, show high *in vivo* activity, as shown in Table IIIA. The compound in which X is 3,5 difluoro, Y is —H, R is —H n is 1 ($IC_{50} = 1.2 \times 10^{-6}$) showed an especially pronounced effect on the DA/NE ration *in vivo.*

In a study on the effect on blood pressure in spontaneously hypertensive rats of daily doses of compounds of the invention (50 mg/kg, i.p.), the compound in which X is 3,5-dichloro, Y is —H, R is —H and n is 1 exhibited a cumulative effect, that is blood pressure continued to decrease on each day of the four four day study period.

The compounds can be incorporated into convenient dosage unit forms such as capsules, tablets or injectable preparations. Pharmaceutical carriers which can be employed can be solid or liquid. Solid carriers include, among others, lactose, terra alba, sucrose, talc, gelatin, agar, pectin, acacia, magnesium stearate, and stearic acid. Liquid carriers include, among others, syrup, peanut oil, olive oil and water. Similarly, the carrier or diluent may include any time delay material, such as glyceryl monostearate or glyceryl distearate, alone or with a wax. The amount of solid carrier will vary widely but, preferably, will be from about 25 mg to about 1 g per dosage unit. If a liquid carrier is used, the preparation will be in the form of a syrup, emulsion, soft gelatin capsule, sterile injectable liquid such as an ampule, or an aqueous or nonaqueous liquid suspension.

The pharmaceutical preparations are made following conventional techniques of a pharmaceutical chemist involving mixing, granulating and compressing, when necessary, for tablet forms, or mixing, filling and dissolving the ingredients, as appropriate, to give the desired oral or parenteral end products.

Doses of the present compounds in a pharmaceutical dosage unit will be an effective amount, that is, a nontoxic quantity selected from the range of 0.1—1,000 mg/kg of active compound, preferably 10—100 mg/kg. The selected dose is administered to a patient in need of treatment from 1—5 times daily, orally, rectally, by injection or by infusion. Parenteral administration, which uses a low dose is preferred. However, oral administration, at a higher dose, can also be used when safe and convenient for the patient. Use of lowest effective doses is recommended because toxicity has been associated with sulfur-containing compounds.

The following examples are illustrative of preparation of compounds of the invention or intermediates therefor. The starting compounds are commercially available or are prepared by known techniques. The Examples are not intended to limit the scope of the invention as defined herein above and as claimed below. The compounds listed in Tables I, II and II, above, were prepared substantially by the illustrated procedures. All temperatures and melting points (mp) are in degrees Celsius (°C).

Example 1

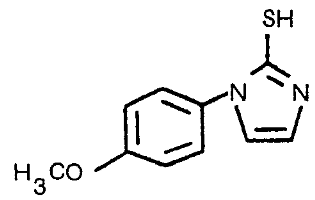

A solution of 10 g (.06 moles) of p-methoxyphenylisothiocyanate in 100 ml of $CHCl_3$ was treated with 6.3 g (.06 moles) of aminoacetaldehyde dimethyl acetal. The solvent was evaporated and the residue was recrystallized from ethanol to yield N-(p-methoxyphenyl)-N′-(ß,ß-dimethoxyethyl)thiourea, 9.2 g (57%). A suspension of this thiourea in a solution of 5 ml of concentrated $H_2SO_4$ and 20 ml of $H_2O$ was refluxed for 3 hr. The mixture was cooled and a solid was filtered, washed with $H_2O$ and dried. Recrystallization from ethanol gave 1-(4-methoxyphenyl)-2-mercaptoimidazole, 4.9 g (70%), mp 215—7°. The compound is deprotected, for example, as illustrated in Example 5 and 6, below, to prepare the phenol, Y is —OH.

Example 2

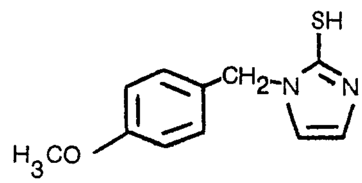

A mixture of 13.6 g (0.1 mole) of anisaldehyde, 13.3 g (0.1 mole) of aminoacetaldehyde diethyl acetal and 1 ml of $CH_3OH$ was heated at 95° for 10 minutes. A residue was dissolved in 150 ml of ethanol and hydrogenated over 10% PD on carbon at 50 psi (0.34 MPa) until $H_2$ uptake was complete. The catalyst was filtered and the filtrate was treated with 10.4 g (0.107 mole) of KSCN, 40 ml of 3N HCl and 40 ml of $H_2O$. The mixture was refluxed, letting the solvent evaporate until the volume of the reaction mixture was 100 ml. After 45 minutes, the mixture was cooled, and a solid was filtered, washed with $H_2O$ and dried. Recrystallization from ethanol gave (1-(4-methoxybenzyl)-2-mercaptoimidazole, 15.0 g (68%), mp 140—142°.

Example 3

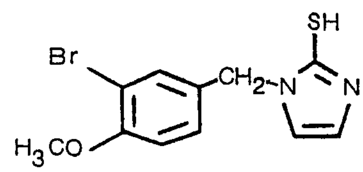

A solution of 10.75 g (.05 mole) of 3-bromo-4-methoxybenzaldehyde and 6.65 g (.05 mole) of aminoacetaldehyde diethyl acetal in 25 ml of ethanol was refluxed for 30 minutes. The solvent was evaporated and the residue was dissolved in $CH_2Cl_2$. The $CH_2Cl_2$ solution of the Schiff base was washed with saturated aqueous NaCl, dried ($K_2CO_3$) and filtered, and the solvent was evaporated. Residual Schiff base was dissolved in 100 ml of methanol, cooled to 5°, and treated with 5.0 g of $NaBH_4$. The reaction mixture was allowed to warm to 22° and, after 4 hr, the solvent was evaporated. The residue was taken up in diethyl ether, washed with $H_2O$, dried ($MgSO_4$) and filtered, and the solvent was evaporated. A solution of the residue in $CHCl_3$, upon treatment with ethereal HCl gave, on standing, crystals of N-(3-bromo-4-methoxybenzyl) aminoacetaldehyde diethylacetal hydrochloride, 10.7 g (58%), mp 112—120°.

A solution of 10.7 g (.029 mole) of N-(3-bromo-4-methoxybenzyl)aminoacetaldehyde diethyl acetal hydrochloride and 3.39 g (0.35 mole) of KSCN in 50 ml of $H_2O$, 50 ml of ethanol and 5 ml of 3N HCl was refluxed for 4.5 hr. One hundred of $H_2O$ was added and the mixture was cooled. A solid was filtered, washed with $H_2O$ and dried. Recrystallization from ethanol gave 1-(3-bromo-4-methoxybenzyl)-2-mercaptoimidazole, 6.3 g (72%) mp 188°.

Example 4

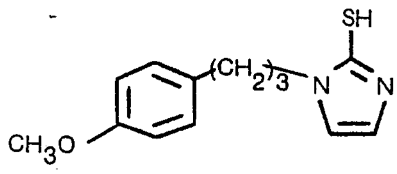

A solution of 12.5 g (.07 mole) of p-methoxyphenylpropionic acid in 100 ml of $CH_2Cl_2$ and one drop of pyridine was treated with 9.8 g (.77 mole) of oxalyl chloride. After 2.5 hr, the solvents were thoroughly evaporated to give the acid chloride as an oil. A solution of the acid chloride in 100 ml of $CH_2Cl_2$ was slowly added to a cold (0°) solution of 14.7 g (0.14 mole) of aminoacetaldehyde dimethyl acetal in 300 ml of $CH_2Cl_2$ at a rate such that the temperature stayed below 20°. After 1 hr, the reaction mixture was poured into $H_2O$, and the $CH_2Cl_2$ layer was separated and washed with aqueous $Na_2CO_3$, 0.5N HCl and $H_2O$. Following drying and evaporation of the solvent, N-(ß,ß-dimethoxyethyl)-p-methoxyphenylpropionamide was left as a solid, 10.3 g (55%). A solution of this amide in 300 ml of diethyl ether was slowly added to a slurry of 4.0 g of $LiAlH_4$ in 400 ml of diethyl ether and 350 ml of tetrahydrofuran (THF). After 3.5 hr at 22°, excess $LiAlH_4$ was cautiously destroyed, the reaction mixture was filtered and the filtrate was evaporated. The residue was dissolved in 100 ml of 0.15N HCl, washed with diethyl ether, basified with $NaHCO_3$ and extracted with diethyl ether. The extracts were dried ($MgSO_4$) and the solvent was evaporated to give N-[3-(4-methoxyphenyl)propyl]aminoacetaldehyde dimethyl acetal, 4.6 g (52%), an an unstable oil.

A solution of 3.62 g (.014 mole) of N-[3-(4-methoxyphenyl)propyl]aminoacetaldehyde dimethyl acetal and 1.4 g (.0144 mole) of KSCN in 20 ml of ethanol, 5 ml of $H_2O$ and 2 ml of concentrated HCl was refluxed for five hr. Fifty ml of $H_2O$ was added, the mixture was cooled and a solid was filtered, washed with $H_2O$ and dried. Recrystallization from ethanol gave 1-[3-(4-methoxyphenyl)-propyl]-2-mercaptoimidazole, 2.4 g (69%), mp 108—109°.

Example 5

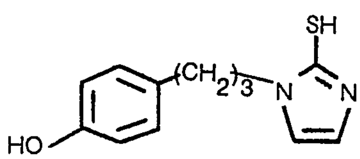

A solution of 1.75 g (.007 mole) fo 1-[3-(4-methoxyphenyl)propyl]-2-mercaptoimidazole in 60 ml of $CH_2Cl_2$ was deprotected by treatment with a solution of 7.0 g (.028 mole) of $BBr_3$ in 10 ml of $CH_2Cl_2$. After 1.5 hr, the reaction mixture was cooled to 0°. and methanol was cautiously added. After a vigorous reaction subsided, the solvents were evaporated. The residue was recrystallized from ethanol to give 1-[3-(4-hydroxyphenyl)propyl]-2-mercaptoimidazole. 1.02 g (67%), mp 185°.

Example 6

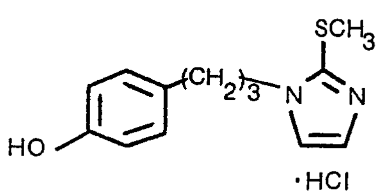

A solution of 1.2 g (.0046 mole) of 1-[3-(4-methoxyphenyl)propyl]-2-mercaptoimidazole in 40 ml of $CH_2Cl_2$ was treated with a solution of 3.5 g (.014 mole) of $BBr_3$ in 10 ml of $CH_2Cl_2$. After 4 hr, methanol was cautiously added, the mixture was stirred for an additional 10 hr, and the solvents were evaporated. The residue was dissolved in $H_2O$, washed with ethyl acetate, neutralized with $NaHCO_3$ and extracted with ethyl acetate. The extracts were dried ($MgSO_4$) and filtered and the solvent was evaporated. The residue was dissolved in 5 ml of ethanol, and treated with ethereal HCl. A crystalline product was filtered and recrystallized from ethanol, to give 1-[3-(hydroxyphenyl)propyl]-2-thiomethylimidazole hydrochloride, 0.61 g (45%), mp 140—142°.

Example 7

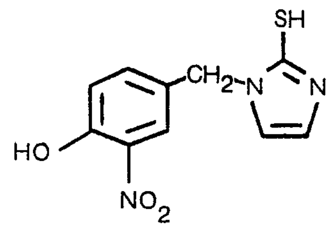

A solution of 1-[3'-nitro-4'-methoxybenzyl]-2-mercaptoimidazole (1.59 g, 6.0 moles) in 10% aqueous NaOH (200 ml) was refluxed for two hr, cooled, acidified with concentrated HCl, cooled and filtered. The crystalline product was washed with water. Recrystallization from ethanol provided 1.25 g (80%) of product as yellow prisms: mp 225—227° (dec).

Example 8

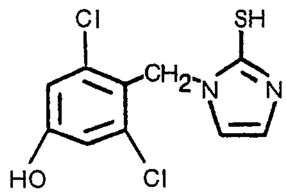

A mixture of 1-[2',6'-dichloro-4-methoxybenzyl]-2-mercaptoimidazole (1 g) in concentrated aqueous hydrobromic acid (50 ml) was heated at reflux under argon for 1.25 hr, and then cooled. The product was collected by filtration. Washing with concentrated aqueous hydrobromic acid and water and drying yielded 0.64 g (60%) of product as light yellow crystals: mp 235° (dec).

Example 9

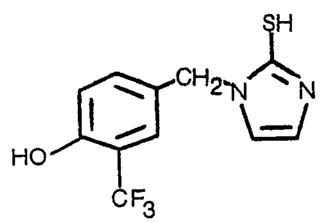

A mixture of 1-[3'-trifluoromethyl-4'-methoxybenzyl]-2-mercaptoimidazole (2.0 g) and pyridine hydrochloride (15 g) was melted at 210° for 30 minutes, cooled, diluted with water and extracted with ethyl acetate. The ethyl acetate extracts were treated with charcoal, dried over sodium sulfate and concentrated to give a thick oil. Addition of THF (3 ml), ether (6 ml), and hexane (15 ml) produced yellow crystals. Recrystallization from ethyl acetate/hexane yielded 0.7 g (36%) of cream coloured crystals: mp 220° (dec).

Example 10

1-[3',5'-Dichlorobenzyl]-2-mercaptoimidazole

A mixture of 3,5-dichlorobenzaldehyde (17.5 g, 0.1 mole) and aminoacetaldehyde diethylacetale (13.3 g, 0.1 mole) was heated on a steam bath. The resulting solution was stirred at room temperature during slow addition of sodium borohydride (3 g, 0.08 mole) and the mixture was stirred overnight at room temperature. The mixture was concentrated under reduced pressure, and the residue was partitioned between ethyl acetate and water. The ethyl acetate layer was washed sequentially with water and brine, and then dried over anhydrous sodium sulfate, and concentrated under reduced pressure. The resulting oil was heated at reflux with water (100 mL), concentrated hydrochloride acid (22 mL), ethanol (41 mL) and potassium thiocyanate (10.7 g, 0.11 mole) for 2 hours. The mixture was cooled and diluted with water (250 mL), and the crude product was collected by filtration and dried. Recrystallization twice from acetic acid provided 7.3 g (30%) of the title compound as light yellow crystals: mp 209—211°.

Example 11

1-[2',2'-Dichlorobenzyl]-2-mercaptoimidazole

Reaction of 2,6-dichlorobenzaldehyde (17.5g, 0.1 mole) and aminoacetaldehyde diethylacetal (13.3 g, 0.1 mole) substantially as above yielded 5.4h (21%) of the title compound as white needles: mp 242—3° (ethanol/ether).

Example 12

1-[2'-Chlorobenzyl]-2-mercaptoimidazole

Reaction of 2-chlorobenzaldehyde (14g, 0.1 mole) and aminoacetaldehyde diethylacetal (13.g, 0.1 mole) substantially as above yielded 11.2g (50%) of the title compound as white crystals: mp 207—7° (acetone/ethanol).

Example 13

1-[2',5'-Dichlorobenzyl]-2-mercaptoimidazole

Reaction of 2,5-dichlorobenzaldehyde (10.25g, 0.059 mole) and aminoacetaldehyde diethylacetal (7.70g, 0.059 mole) substantially as above yielded 5.2g (34%) of the title compounds as white crystals: mp 265° (dec).

Example 14

1-[4'-Chlorobenzyl]-2-mercaptoimidazole

Reaction of 4-chlorobenzaldehyde (14g, 0.1 mole) and aminoacetaldehyde diethylacetal (13.3g, 0.1 mole) substantially as above yielded 8.5g (36%) of the title compound as white crystals: mp 187—9° (acetonitrile).

Example 15

1-[2',3'-Dichlorobenzyl]-2-mercaptoimidazole

Reaction of 2,3-dichlorobenzaldehyde (8.7g, 0.05 mole) and aminoacetaldehyde diethylacetal (6.65g, 0.05 mole) substantially as above yielded 3.0g (23%) of the title compound as white crystals: mp 196—7° (ethanol).

Example 16

1-[4'-Fluorobenzyl]-2-mercaptoimidazole

Reaction of 4-fluorobenzaldehyde (12.4g, 0.1 mole) and aminoacetaldehyde diethylacetal (13.3g, 0.1 mole) substantially as above yielded 13.0g (62.5%) of the title compound as white crystals. mp 167—9° (ethanol).

Example 17

1-[3',4'-Dichlorobenzyl]-2-mercaptoimidazole

Reaction of 3,4-dichlorobenzaldehyde (17.5g, 0.1 mole) and aminoacetaldehyde diethylacetal (13.3g, 0.1. mole) substantially as above yielded 10g (39%) of the title compound as white crystals: mp 178—81° (ethanol).

Example 18

1-[2',4'-Dichlorobenzyl]-2-mercaptoimidazole

Reaction of 2,4-dichlorobenzaldehyde (17.5g, 0.1 mole) and aminoacetaldehyde diethylacetal (13.3g, 0.1 mole) substantially as above yielded 8.5g (33%) of the title compound as of white crystals: mp 185—7° (2-propanol).

Example 19

1-[3'-Chlorobenzyl]-2-mercaptoimidazole

Reaction of 3-chlorobenzaldehyde (14g, 0.1 mole) and aminoacetaldehyde diethylacetal (13.3g, 0.1 mole) substantially as above yielded 14g (62.5%) of the title compound as white crystals: mp 129—131° (acetonitrile).

Example 20

1-[2',4',6'-Trichlorobenzyl]-2-mercaptoimidazole

Reaction of 2,4,6-trichlorobenzaldehyde (20.9g, 0.1 mole and aminoacetaldehyde diethylacetal (13.2g, 0.1 mole) substantially as above yielded 12g (46%) of the title compound as white crystals: mp 240—4° (ethanol).

Example 21

1-[3-Fluorobenzyl]-2-mercaptoimidazole

Reaction of 3-fluorobenzaldehyde (24.8g, 0.2 mole) and aminoacetaldehyde diethyl acetal (26.6g, 0.2 mole) substantially as above yielded 28g (67%) of the title compound as white crystals: mp 11—114° (2-propanol/water).

Example 22

1-[3',5'-Difluorobenzyl]-2-mercaptoimidazole

Reaction of 3,5-difluorobenzaldehyde (14.7g, 0.014 mole) and aminoacetaldehyde dimethyl acetal (10.8g, 0.014 mole) substantially as above yielded 10.0g (43%) of the title compound as white crystals: mp 140—1° (ethyl acetate/hexane).

Example 23

1-[3-(3',5'-Difluorophenyl)propyl]-2-mercaptoimidazole

3,5-Difluorobenzaldehyde (5.5 g, 0,039 mole), malonic acid (6.06 g, 0.058 mole), pyridine (2.1 ml) and piperidine (0.105 ml) were heated on a steam bath for 2 hr and then at 155° for 1 hr. The reaction mixture was poured into cold 3N aqueous hydrochloric acid, and then filtered. Recrystallisation from ethanol provided 4.7 g (66%) of 3,5-difluorocinnamic acid as buff needles: mp 199—201°C.

3,5-Difluorocinnamic acid (4.6 g, 0.025 mole) was dissolved in tetrahydrofuran (50 ml) and added to a slurry of 0.75 g palladium/carbon in ethyl acetate. The mixture was shaken under 50 psi (.34 MPa) hydrogen for 5 hr and then was filtered and concentrated to provide 4.5g (97%) of 3-[3',5'-difluorophenyl] propanoic acid as colorless crystals: mp 56° (methanol).

A solution of 3-[3',5'-difluorophenyl] propanoic acid (4.4 g, 0.024 mole) N,N-dimethylformamide (one drop) and thionyl chloride (15 ml) was heated at 60° for 3 hr. Excess thionyl chloride was removed by distillation at reduced pressure. Distillation (Kugelrohr) at reduced pressure (about 0.25 mm (33 Pa)) yielded 4.1 g (85%) of 3-[3',5'-difluorophenyl]propionyl chloride as an oil.

A solution of 3-[3',5'-difluorophenyl]propionyl chloride (4 g, 0.0196 mole) in methylene (40 ml) was slowly added to a 0° solution of amino acetaldehyde dimethylacetal (4.3 g, 0.0412 mole) in methylene chloride (100 ml) at a rate such that the temperature did not exceed 20°. The reaction mixture was stirred for 1 hr. Then it was poured into water and the layers were separated. The organic layer was washed with 5% aqueous sodium carbonate, 0.05% aqueous hydrogen chloride, and water and then was dried over sodium sulfate and concentrated to yield 5.5 g (103%) 3-[3',5'-difluorophenyl]propanamide N-acetaldehyde dimethyl acetal as an oil.

A solution of 3-[3',5'-difluorophenyl]propanamide N-acetaldehyde dimethyl acetal (5.3 g, 0.0194 mole) in diethyl ether (100 ml) was slowly added to a slurry of lithium aluminum hydrided (4.4 g, 0.116 mole) in diethyl ether (200 ml). The reaction mixture was stirred at ambient temperature (20—25°) for 18 hr. Then water (4.5 ml) was carefully added, followed by 10% aqueous sodium hydroxide (7 ml) and water (11 ml). The mixture was filtered and the filtrate was dried over sodium sulfate and concentrated to yield 4.4 g (88%) of 3-[3',5'-difluorophenyl]propanamine N-acetaldehyde dimethylacetal as colorless oil.

A solution of 3-[3',5'-difluorophenyl]propanamine N-acetaldehyde dimethyl acetal (4.3 g, 0.0166 mole) and potassium thiocyanate (1.6 g, 0.0166 mol) in ethanol (12 ml), water (20 ml), and concentrated hydrochloric acid (4 ml) was refluxed for 1 hr and then cooled and a large volume of water was added. The product was filtered and recrystallized to yield 2.2 g (55%) of 1-[3-(3',5'-difluorophenyl)propyl]-2-mercaptoimidazole as white needles: mp 131—132° (ethanol).

Example 24

3-(3',5'-Dichlorophenyl)propyl-2-mercaptoimidazole

Reaction of 3,5-dichlorobenzadehyde (26.9g, 0.154 mole), malonic acid (24.1 g, 0.232 mole), pyridine (8 ml) and piperidine (0.4 ml) substantially as above yielded 22.9 g (69%) of 3,5-dichlorocinnamic acid as white needles: mp 169—170° (ethanol).

Reaction of 3,5-dichlorocinnamic acid (22.9 g, 0.106 mol) and 3 g palladium/carbon substantially as above yielded 23 g (99%) of 3-[3',5'-dichlorophenyl]propanoic acid as an oil.

A one molar solution of borane in tetrahydrofuran (233 ml) was added dropwise to a cooled (0°) solution of 3-(3',5'-dichlorophenyl)propanoic acid (23 g, 0.106 mole) in distilled tetrahydrofuran (200 ml). The reaction was stirred at room temperature for 2 hr. Then methanol was added and the solution was concentrated to yield 21.2 g (98%) of 1-[3-(3',5'-dichlorophenyl)]propanol as a clear oil.

Dimethyl sulfoxide (6.75 g, 0.083 mole) in dry methylene chloride (15 ml) was added dropwise to a solution of oxalylchloride (6.2 g, 0.049 mole) in dry methylene chloride (15 ml) at −78°. The reaction mixture was stirred for 2 min. Then 1-[3-(3',5'-dichlorophenyl)]propanol(5 g, 0.0245 mole) in dry methylene chloride (20 ml) was slowly added, keeping the temperature below −60°. After stirring for 15 min at −70°, triethylamine (16 g, 0.160 mol) was added dropwise. The reaction mixture was stirred for an additional 5 min at −60° and then was warmed to room temperature and diluted with water. The organic layer was separated, washed with 3N aqueous hydrogen chloride and then with brine, and was dried over sodium sulfate. The solution was concentrated to give 5.0 g (100%) of 3-(3',5'-dichlorophenyl)propionaldehyde as a yellow oil.

Amino acetaldehyde dimethylacetal (2.1 g, 0.0197 mole) was added with stirring to a solution of 3-(3',5'-dichlorophenyl)propionaldehyde (5 g, 0.025 mole) in hexane (10 ml). After stirring for 1 hr at room temperature, sodium borohydride (7.3 g, 0.193 mole) in ethanol (25 ml) was added. The reaction mixture was stirred for 18 hr and then was diluted with water and concentrated. The residue was taken up in ethylacetate, washed with water, dried over sodium sulfate and concentrated to yield 6.8 g (93%) of 3-(3',5'-dichlorophenyl)propanamide N-acetaldehyde dimethyl acetal as a yellow oil.

A solution of 3-(3',5'-dichlorophenyl)propanamide N-acetaldehyde dimethyl acetal and potassium thiocynate (2.2 g, 0.0223 mole) in ethanol (20 ml), water (30 ml), and concentrated hydrochloric acid was refluxed for 1 hr. The reaction was cooled and diluted with water. After standing for 3 hr, the crude product solidified and was filtered. Chromatography on silica, eluting with 0.5 to 1% methanol in methylene chloride, provided 2.0 g (31%) of 3-(3',5'-dichlorophenyl)propyl-2-mercaptoimidazole as white crystls: mp 98—99° (ethanol).

**Claims for the Contracting States: BE CH DE FR GB IT LI LU NL SE**

1. A compound having the formula:

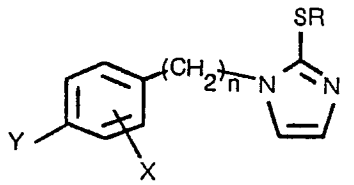

wherein

X is —H, —OH, halogen, $C_{1-4}$alkyl, —CN, —$NO_2$, —$SO_2NH_2$, —$CO_2H$, —$CONH_2$, —CHO, —$CH_2OH$, —$CF_3$, —$OCH_3$, —$SO_2C_{1-4}$alkyl, —$SO_2C_{1-4}$fluoroalkyl, or —$CO_2C_{1-4}$alkyl or any synthetically accessible combination thereof up to four substituents;

Y is —H, —OH, halogen, $C_{1-4}$alkyl, —CN, —$NO_2$, —$SO_2NH_2$, —$CO_2H$, —$CONH_2$, —CHO, —$CH_2OH$, —$CF_3$, —$SO_2C_{1-4}$alkyl, —$SO_2C_{1-4}$fluoroalkyl, or —$CO_2C_{1-4}$alkyl; and

R is —H or $C_{1-4}$alkyl; and,

n is 0—4,

or a hydrate or, when R is $C_{1-4}$alkyl, a pharmaceutically acceptable acid addition salt thereof, provided that when n is 0, Y is —OH and when n is 1—3, at least one of Y and X is not —H.

2. The compound of claim 1 in which X is a single substituent.

3. The compound of claim 1 which is

1-(4-hydroxyphenyl)-2-mercaptoimidazole
1-(4-hydroxybenzyl)-2-mercaptoimidazole
1-[2-(4-hydroxyphenyl)ethyl]-2-mercaptoimidazole
1-[3-(4-hydroxyphenyl)propyl]-2-mercaptoimidazole
1-(3,4-dihydroxybenzyl)-2-mercaptoimidazole
1-(3-chloro-4-hydroxybenzyl)-2-mercaptoimidazole
1-(3-methyl-4-hydroxybenzyl)-2-mercaptoimidazole
1-(3-bromo-4-hydroxybenzyl)-2-mercaptoimidazole
1-(3-fluoro-4-hydroxybenzyl)-2-mercaptoimidazole
1-(3-nitro-4-hydroxybenzyl)-2-mercaptoimidazole
1-(3-trifluoromethyl-4-hydroxybenzyl)-2-mercaptoimidazole
1-(2,6-dichloro-4-hydroxybenzyl)-2-mercaptoimidazole
1-(2,3,4,6-tetrafluoro-4-hydroxybenzyl)-2-mercaptoimidazole
1-[3-(4-hydroxyphenyl)propyl]-2-thiomethylimidazole
1-(4-fluorobenzyl)-2-mercaptoimidazole
1-(2,6-dichlorobenzyl)-2-mercaptoimidazole
1-(2-chlorobenzyl)-2-mercaptoimidazole
1-(2,5-dichlorobenzyl)-2-mercaptoimidazole
1-(2,3-dichlorobenzyl)-2-mercaptoimidazole
1-(3-chlorobenzyl)-2-mercaptoimidazole
1-(3,5-dichlorobenzyl)-2-mercaptoimidazole
1-(3-fluorobenzyl)-2-mercaptoimidazole
1-(3,5-difluorobenzyl)-2-mercaptoimidazole
1-(4-chlorobenzyl)-2-mercaptoimidazole
1-(3,4-dichlorobenzyl)-2-mercaptoimidazole
1-(2,4-dichlorobenzyl)-2-mercaptoimidazole
1-(2,4,6-dichlorobenzyl)-2-mercaptoimidazole
1-[3-(3,5-dichlorophenyl)propyl]-2-mercaptoimidazole
1-[3-(3,5-difluorophenyl)propyl]-2-mercaptoimidazole
1-(3-hydroxybenzyl)-2-mercaptoimidazole
1-(2-hydroxybenzyl)-2-mercaptoimidazole
1-(2-methoxybenzyl)-2-mercaptoimidazole
1-(3-methoxybenzyl)-2-mercaptoimidazole
1-benzyl-2-mercaptoimidazole
1-(3,5-difluorobenzyl)-2-mercaptoimidazole.

4. A pharmaceutical composition comprising a compound having the formula:

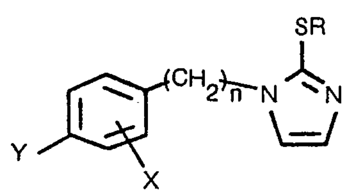

wherein X, Y, R and n are as defined in claim 1 or a hydrate or, when R is $C_{1-4}$alkyl, a pharmaceutically acceptable acid addition salt thereof, provided that when n is 0, Y is —OH, and a suitable carrier.

5. The pharmaceutical composition of claim 4 wherein X is a single substituent.

6. The pharmaceutical composition of claim 4 wherein the compound is a compound according to claim 3.

7. A compound of formula,

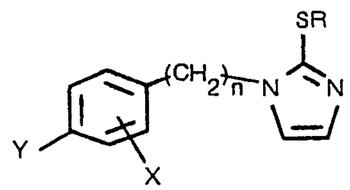

wherein X, Y, R and n is as defined in claim 1 or a hydrate or, when R is $C_{1-4}$alkyl, a pharmaceutically acceptable acid addition salt thereof for use as an active pharmaceutical substance.

8. A compound of claim 7 wherein X is a single substituent for use as an active pharmaceutical substance.

9. A compound of claim 7 which is a compound according to claim 3 for use as an active pharmaceutical substance.

10. A compound as claimed in any one of claims 7—9, for use in the inhibition of dopamine β-hydroxylase activity in mammals.

11. A process for preparing a compound of claim 1, which comprises:

(a) reaction of a compound having the formula:

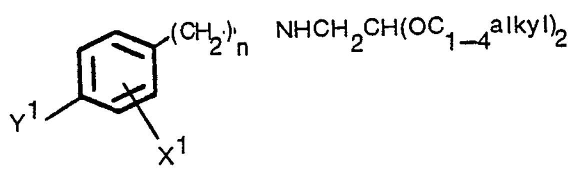

wherein $X^1$is X, but not OH, $Y^1$ is Y, but not OH and n is 0—4 with acidic thiocyanate; or

(b) wherein the compound of claim 1, n is 0, cyclisation of a compound of formula:

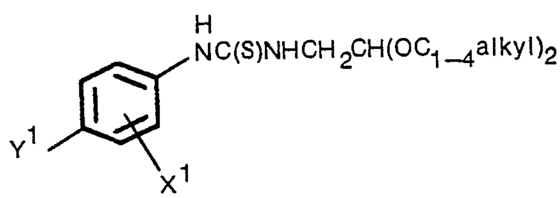

wherein $Y^1$ is $OCH_3$ and $X^1$ is as defined in (a) above, in the presence of an acid; and optionally alkylating the compound of claim 1 so formed where R is —H to a compound of claim 1 wherein R is $C_{1-4}$alkyl and/or, where $X^1$ and/or $Y^1$ are —$OCH_3$, optionally deprotecting the methoxy group(s) to prepare a compound of claim 1 wherein X and/or Y are —OH.

**Claims for the Contracting State: AT**

1. A process for the preparation of a compound of formula,

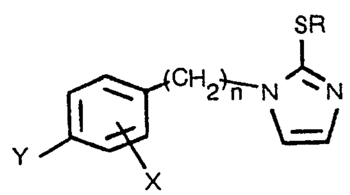

wherein

X is —H, —OH, halogen, $C_{1-4}$alkyl, —CN, —$NO_2$, —$SO_2NH_2$, —$CO_2H$, —$CONH_2$, —CHO, —$CH_2OH$, —$CF_3$, —$OCH_3$, —$SO_2C_{1-4}$alkyl, —$SO_2C_{1-4}$fluoroalkyl, or —$CO_2C_{1-4}$alkyl or any synthetically accessible combination thereof up to four substituents;

Y is —H, —OH, halogen, $C_{1-4}$alkyl, —CN, —$NO_2$, —$SO_2NH_2$, —$CO_2H$, —$CONH_2$, —CHO, —$CH_2OH$, —$CF_3$, —$SO_2C_{1-4}$alkyl, —$SO_2C_{1-4}$fluoroalkyl, or —$CO_2C_{1-4}$alkyl; and

R is —H or $C_{1-4}$alkyl; and,

n is 0—4,

or a hydrate or, when R is $C_{1-4}$alkyl, a pharmaceutically acceptable acid addition salt thereof, provided that when n is 0, Y is —OH and when n is 1—3, at least one of Y and X is not —H, which comprises,

(a) reacting a compound of formula,

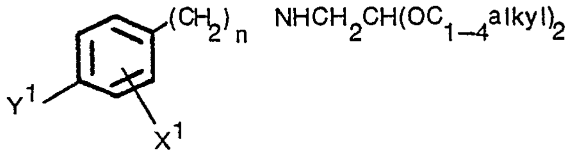

wherein $Y^1$ is Y but not —OH, and $X^1$ is X, but not —OH, and n is 0—4, with acidic thiocyanate; or,

(b) where n is 0, cyclisation of a compound of formula,

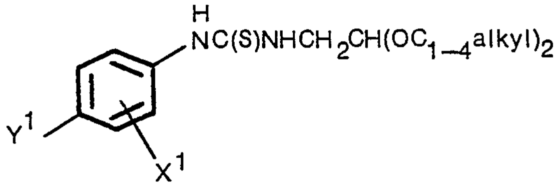

wherein $Y^1$ is —$OCH_3$ and $X^1$ is as defined in (a) above in the presence of an acid; and, optionally alkylating the compound so formed where R is —H, to a compound where R is $C_{1-4}$alkyl, and/or, where $X^1$ and/or $Y^1$ are —$OCH_3$, optionally deprotecting the methoxy group(s) to prepare a compound wherein X and/or Y are —OH.

2. A process for the preparation of a pharmaceutical composition which comprises combining a compound of structure,

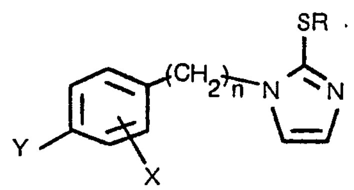

wherein X, Y, R and n are as defined in claim 1 or a hydrate or, when R is $C_{1-4}$alkyl, a pharmaceutically acceptable acid addition salt thereof when n is 0, Y is OH with a pharmaceutically acceptable carrier therefor.

**Patentansprüche für die Vertragsstaaten: BE CH DE FR GB IT LI LU NL SE**

1. Verbindung der Formel:

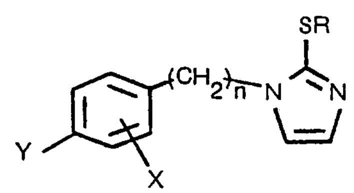

in der

X —H, —OH, Halogen, $C_{1-4}$-Alkyl, —CN, —$NO_2$, —$SO_2NH_2$, —$CO_2H$, —$CONH_2$, —CHO, —$CH_2OH$, —$CF_3$, —$OCH_3$, —$SO_2$—$C_{1-4}$-Alkyl, —$SO_2$—$C_{1-4}$-Fluoralkyl oder —$CO_2$—$C_{1-4}$-Alkyl oder irgendeine synthetisch zugängliche Kombination davonmit bis zu 4 Substituenten bedeutet;

Y H—, —OH, Halogen, $C_{1-4}$-Alkyl, —CN, —$NO_2$, —$SO_2NH_2$, —$CO_2H$, —$CONH_2$, —CHO, —$CH_2OH$, —$CF_3$, —$SO_2$—$C_{1-4}$-Alkyl, —$SO_2$—$C_{1-4}$-Fluoralkyl oder —$CO_2$—$C_{1-4}$-Alkyl bedeutet;

R H- oder $C_{1-4}$-Alkyl bedeutet; und

n Werte von 0 bis 4 hat, oder ein Hydrat oder, wenn R $C_{1-4}$ Alkyl ist, ein pharmazeutisch verträgliches Säureadditionssalz davon, mit der Maßgabe, daß Y —OH bedeutet, wenn n den Wert Null hat und mindestens einer der Reste Y oder X nicht —H bedeutet, wenn n Werte von 1 bis 3 hat.

2. Verbindung nach Anspruch 1, in der X ein einziger Substituent ist.

3. Verbindung nach Anspruch 1, nämlich:
1-(4-Hydroxyphenyl)-2-mercaptoimidazol,
1-(4-Hydroxybenzyl)-2-mercaptoimidazol,
1-[2-(4-Hydroxyphenyl)-äthyl]-2-mercaptoimidazol,
1-[3-(4-Hydroxyphenyl)-propyl]-2-mercaptoimidazol,
1-(3,4-Dihydroxybenzyl)-2-mercaptoimidazol,
1-(3-Chlor-4-hydroxybenzyl)-2-mercaptoimidazol,
1-(3-Methyl-4-hydroxybenzyl)-2-mercaptoimidazol,
1-(3-Brom-4-hydroxybenzyl)-2-mercaptoimidazol,
1-(3-Fluor-4-hydroxybenzyl)-2-mercaptoimidazol,
1-(3-Nitro-4-hydroxybenzyl)-2-mercaptoimidazol,
1-(3-Trifluormethyl-4-hydroxybenzyl)-2-mercaptoimidazol,
1-(2,6-Dichlor-4-hydroxybenzyl)-2-mercaptoimidazol,
1-(2,3,4,6-Tetrafluor-4-hydroxybenzyl)-2-mercaptoimidazol,
1-[3-(4-Hydroxyphenyl)-propyl]-2-thiomethylimidazol,
1-(4-Fluorbenzyl)-2-mercaptoimidazol,
1-(2,6-Dichlorbenzyl)-2-mercaptoimidazol,
1-(2-Chlorbenzyl)-2-mercaptoimidazol,
1-(2,5-Dichlorbenzyl)-2-mercaptoimidazol,
1-(2,3-Dichlorbenzyl)-2-mercaptoimidazol,
1-(3-Chlorbenzyl)-2-mercaptoimidazol,
1-(3,5-Dichlorbenzyl)-2-mercaptoimidazol,
1-(3-Fluorbenzyl)-2-mercaptoimidazol,
1-(3,5-Difluorbenzyl)-2-mercaptoimidazol,
1-(4-Chlorbenzyl)-2-mercaptoimidazol,
1-(3,4-Dichlorbenzyl)-2-mercaptoimidazol,
1-(2,4-Dichlorbenzyl)-2-mercaptoimidazol,
1-(2,4,6-Trichlorbenzyl)-2-mercaptoimidazol,
1-[3-(3,5-Dichlorphenyl)-propyl]-2-mercaptoimidazol,
1-[3-(3,5-Difluorphenyl)-propyl]-2-mercaptoimidazol,
1-(3-Hydroxybenzyl)-2-mercaptoimidazol,
1-(2-Hydroxybenzyl)-2-mercaptoimidazol,
1-(2-Methoxybenzyl)-2-mercaptoimidazol,
1-(3-Methoxybenzyl)-2-mercaptoimidazol,
1-Benzyl-2-mercaptoimidazol, oder
1-(3,5-Difluorbenzyl)-2-mercaptoimidazol.

4. Arzneimittel, umfassend eine Verbindung der Formel

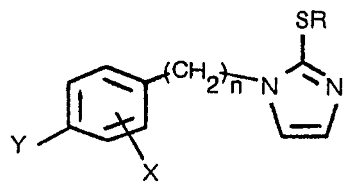

wobei X, Y, R und n wie in Anspruch 1 definiert sind, oder ein Hydrat oder, wenn R $C_{1-4}$-Alkyl ist, ein pharmazeutisch verträgliches Säureadditionssalz davon, mit der Maßgabe, daß Y —OH bedeutet, wenn n den Wert 0 hat und mindestens einer der Reste Y oder X nicht —H bedeutet, wenn n Werte von 1 bis 3 hat.

5. Arzneimittel nach Anspruch 4, wobei X ein einziger Substituent ist.

6. Arzneimittel nach Anspruch 4, wobei die Verbindung eine Verbindung nach Anspruch 3 ist.

7. Verbindung der Formel:

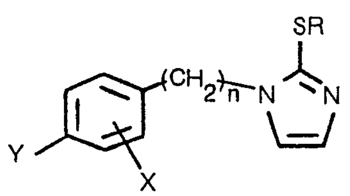

wobei X, Y, R und n wie in Anspruch 1 definiert sind, oder ein Hydrat, oder, wenn R $C_{1-4}$-Alkyl ist, ein pharmazeutisch verträgliches Säureadditionssalz davon, mit der Maßgabe, daß Y —OH bedeutet, wenn n den Wert Null hat und mindestens einer der Reste Y oder X nicht —H bedeutet, wenn n Werte von 1 bis 3 hat.

8. Verbindung nach Anspruch 7, in der X ein einziger Substituent ist zur Verwendung als pharmazeutischer Wirkstoff.

9. Verbindung nach Anspruch 7, die eine Verbindung nach Anspruch 3 ist, zur Verwendung als pharmazeutischer Wirkstoff.

10. Verbindung nach einem der Anspruch 7 bis 9 zur Verwendung bei der Hemmung der Aktivatät von Dopamin-β-hydroxylase bei Säugern.

11. Verfahren zur Herstellung einer Verbindung nach Anspruch 1, welches umfaßt:

(a) Umsetzung einer Verbindung der Formel:

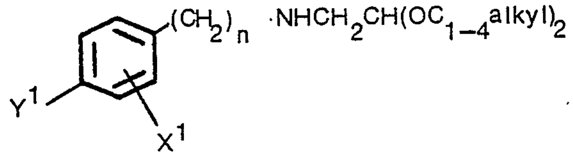

in der $X^1$ X, aber nicht OH und $Y^1$ Y, aber nicht —OH bedeuten und n Werte von Null bis 4 hat, mit saurem Thiocyanat; oder

(b) wenn in der Verbindung nach Anspruch 1, n den Wert Null hat, Cyclisierung einer Verbindung der Formel:

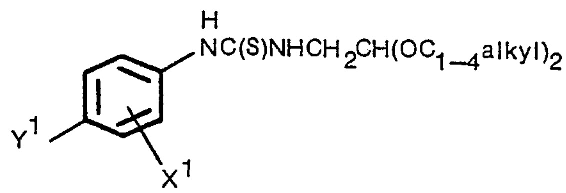

in der $Y^1$ $OCH_3$ bedeutet und $X^1$ wie vorstehend in (a) definiert ist, in Gegenwart einer Säure; und gegebenenfalls Alkylierung der so erhaltenen Verbindung nach Anspruch 1, in der R —H bedeutet, zu einer Verbindung nach Anspruch 1, in der R $C_{1-4}$-Alkyl ist und/oder wenn $X^1$ und/oder $Y^1$-$OCH_3$ bedeuten, gegebenenfalls Abspalten der Methoxygruppe(n) zur Herstellung einer Verbindung nach Anspruch 1, in der X und/oder Y —OH bedeuten.

**Patentansprüche für den Vertragsstaat: AT**

1. Verfahren zur Herstellung einer Verbindung der Formel,

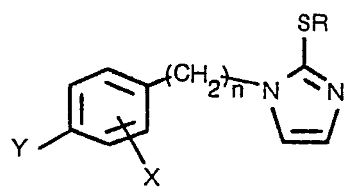

in der

X —H, —OH, Halogen, $C_{1-4}$-Alkyl, —CN, —$NO_2$, —$SO_2NH_2$, —$CO_2H$, —$CONH_2$, —CHO, —$CH_2OH$, —$CF_3$, —$OCH_3$, —$SO_2$—$C_{1-4}$-Alkyl, —$SO_2$—$C_{1-4}$-Fluoralkyl oder —$CO_2$—$C_{1-4}$-Alkyl oder irgendeine synthetisch zugängliche Kombination davon mit bis zu 4 Substituenten bedeutet;

Y H—, —OH, Halogen, $C_{1-4}$-Alkyl, —CN, —$NO_2$, —$SO_2NH_2$, —$CO_2H$, —$CONH_2$, —CHO, —$CH_2OH$, —$CF_3$, —$SO_2$—$C_{1-4}$-Alkyl, —$SO_2$—$C_{1-4}$-Fluoralkyl oder —$CO_2$—$C_{1-4}$-Alkyl bedeutet;

R H- oder $C_{1-4}$-Alkyl bedeutet; und n Werte von 0 bis 4 hat, oder ein Hydrat oder, wenn R $C_{1-4}$ Alkyl ist, ein pharmazeutisch verträgliches Säureadditionssalz davon, mit der Maßgabe, daß Y —OH bedeutet, wenn n den Wert Null hat und mindestens einer der Reste Y oder X nicht —H bedeutet, wenn n Werte von 1 bis 3 hat, umfassend

(a) Umsetzung einer Verbindung der Formel,

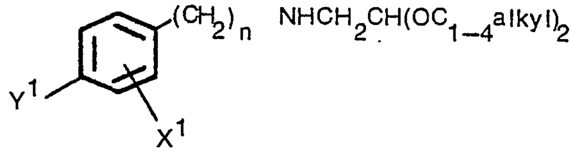

in der $X^1$ X, aber nicht —OH und $Y^1$ Y, aber nicht —OH bedeuten und n Werte von Null bis 4 hat, mit saurem Thiocyanat: oder

(b) wenn n den Wert Null hat, Cyclisierung einer Verbindung der Formel,

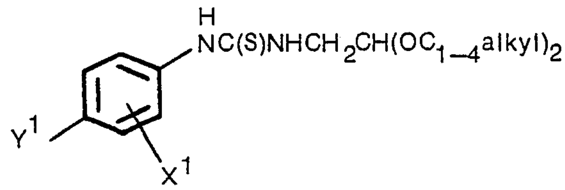

in der $Y^1$ —$OCH_3$ bedeutet und $X^1$ wie vorstehend in (a) definiert ist, in Gegenwart einer Säure; und gegebenenfalls Alkylierung der so erhaltenen Verbindung, in der R —H bedeutet, zu einer Verbindung, in der R $C_{1-4}$-Alkyl ist und/oder wenn $X^1$ und/oder $Y^1$ —$OCH_3$ bedeuten, gegebenenfalls Abspalten der Methoxygruppe(n) zur Herstellung einer Verbindung, in der X und/oder Y —OH bedeuten.

2. Verfahren zur Herstellung eines Arzneimittels, umfassend das Zusammenbringen einer Verbindung der Struktur

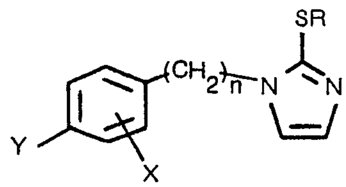

in der X, Y, R und n wie in Anspruch 1 definiert sind, oder eines Hydrates oder, wenn R $C_{1-4}$-Alkyl ist, eines pharmazeutisch verträglichen Säureadditionssalzes davon, mit der Maßgabe, daß Y —OH bedeutet, wenn n den Wert 0 hat, mit einem pharmazeutisch verträglichen Träger dafür.

**Revendications pour les Etats contractants: BE CH DE FR GB IT LI LU NL SE**

1. Composé de formule

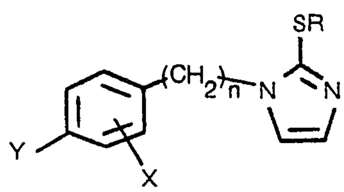

X est —H, —OH, halogène, $C_{1-4}$alkyle, —CN, —$NO_2$, —$SO_2NH_2$, $CO_2H$, —$CONH_2$, —CHO, —$CH_2OH$, —$CF_3$, —$OCH_3$, —$SO_2C_{1-4}$alkyle, —$SO_2C_{1-4}$fluoroalkyle, ou —$CO_2C_{1-4}$alkyle ou toute combinaison synthétiquement accessible de celui-ci, jusqu-à substituants

Y est —H, —OH, halogène, $C_{1-4}$alkyle, —CN, —$NO_2$, —$SO_2NH_2$, —$CO_2H$, —$CONH_2$, —CHO, —$CH_2OH$, —$CF_3$, —$SO_2C_{1-4}$alkyle, —$SO_2C_{1-4}$fluoroalkyle, ou —$CO_2C_{1-4}$alkyle; et .

R est —H ou $C_{1-4}$alkyle et

n est 0—4,

ou un hydrate ou, lorsque R est $C_{1-4}$alkyle, un sel d'addition avec un acide pharmaceutiquement acceptable de celui-ci, à condition que, lorsque n est O, Y soit —OH et, lorsque n est 1—3, au moins un de Y et X ne soit pas —H.

2. Composé de la revendication 1 dans lequel X est un substituant unique.

3. Composé de la revendication 1 qui est
1-(4-hydroxyphényl)-2-mercaptoimidazole
1-(4-hydroxybenzyl)-2-mercaptoimidazole
1-[2-(4-hydroxyphényl)éthyl]-2-mercaptoimidazole
1-[3-(4-hydroxyphènyl)propyl]-2-mercaptoimidazole
1-(3,4-dihydroxybenzyl)-2-mercaptoimidazole
1-(3-chloro-4-hydroxybenzyl)-2-mercaptoimidazole
1-(3-méthyl-4-hydroxybenzyl)-2-mercaptoimidazole
1-(3-bromo-4-hydroxybenzyl)-2-mercaptoimidazole
1-(3-fluoro-4-hydroxybenzyl)-2-mercaptoimidazole
1-(3-nitro-4-hydroxybenzyl)-2-mercaptoimidazole
1-(3-trifluorométhyl-4-hydroxybenzyl)-2-mercaptoimidazole
1-(2,6-dichloro-4-hydroxybenzyl)-2-mercaptoimidazole
1-(2,3,4,6-tétrafluoro-4-hydroxybenzyl)-2-mercaptoimidazole

1-[3-(4-hydroxyphényl)propyl]-2-thiométhylimidazole
1-(4-fluorobenzyl)-2-mercaptoimidazole
1-(2,6-dichlorobenzyl)-2-mercaptoimidazole
1-(2-chlorobenzyl)-2-mercaptoimidazole
1-(2,5-dichlorobenzyl)-2-mercaptoimidazole
1-(2,3-dichlorobenzyl)-2-mercaptoimidazole
1-(3-chlorobenzyl)-2-mercaptoimidazole
1-(3,5-dichlorobenzyl)-2-mercaptoimidazole
1-(3-fluorobenzyl)-2-mercaptoimidazole
1-(3,5-difluorobenzyl)-2-mercaptoimidazole
1-(4-chlorobenzyl)-2-mercaptoimidazole
1-(3,4-dichlorobenzyl)-2-mercaptoimidazole
1-(2,4-dichlorobenzyl)-2-mercaptoimidazole
1-(2,4,6-dichlorobenzyl)-2-mercaptoimidazole
1-[3-(3,5-dichlorobenzyl)propyl]-2-mercaptoimidazole
1-[3-(3,5-difluorophényl)propyl]-2-mercaptoimidazole
1-(3-hydroxybenzyl)-2-mercaptoimidazole
1-(2-hydroxybenzyl)-2-mercaptoimidazole
1-(2-méthoxybenzyl)-2-mercaptoimidazole
1-(3-méthoxybenzyl)-2-mercaptoimidazole
1-benzyl-2-mercaptoimidazole
1-(3,5-difluorobenzyl)-2-mercaptoimidazole.

4. Composition pharmaceutique comprenant un composé de formule

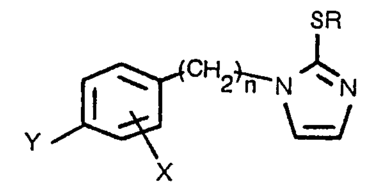

dans laquelle X, Y, R et n sont tels que définis dans la revendication 1 ou un hydrate ou lorsque R est $C_{1-4}$alkyle, un sel d'addition avec un acide pharmaceutiquement acceptable de celui-ci, à condition que, lorsque n est 0, Y soit —OH, et un excipient convenable.

5. Composition pharmaceutique de la revendication 4 dans laquelle X est un substituant unique.

6. Composition pharmaceutique de la revendication 4 dans laquelle le composé est un composé suivant la revendication 3.

7. Composé de formule

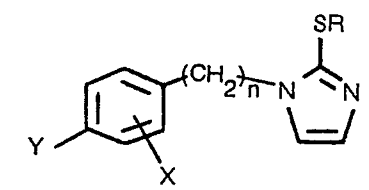

où X, Y, R et n sont tels que définis dans la revendication 1 ou un hydrate ou, lorsque R est $C_{1-4}$alkyle, un sel d'addition avec un acide pharmaceutiquement acceptable de celui-ci pour usage comme substance pharmaceutiquement active.

8. Composé de la revendication 7 où X est un substituant unique, pour usage comme substance pharmaceutiquement active.

9. Composé de la revendication 7 qui est un composé suivant la revendication 3, pour usage comme substance pharmaceutiquement active.

10. Composé tel que revendiqué dans l'une quelconque des revendications 7—9, pour usage dans l'inhibition de l'activité de la dopamine-β-hydroxylase chez les mammifères.

11. Procédé pour préparer un composé de la revendication 1 qui comprend:

(a) la réaction d'un composé de formule:

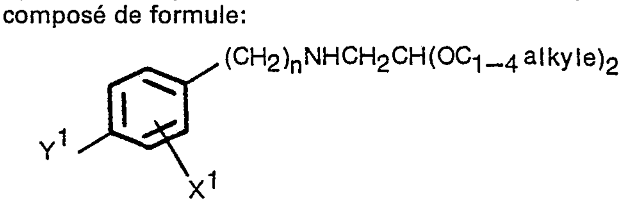

où $X^1$ est X, mais pas OH, $Y^1$ est Y, mais pas OH et n est 0—4 avec un thiocyanate acide ou

(b) lorsque, dans le composé de la revendication 1, n est 0, la cyclisation d'un composé de formule:

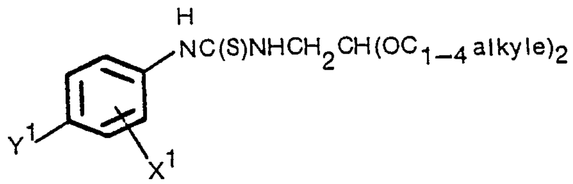

où $Y^1$ est $OCH_3$ et $X^1$ est tel que défini dans (a) ci-dessus, en présence d'un acide; et, si on le désire, l'alkylation du composé de la revendication 1 ainsi formé où R est —H en un composé de la revendication 1 où R est $C_{1-4}$alkyle et/ou, lorsque $X^1$ et/ou $Y^1$ sont —$OCH_3$, si on le désire, la déprotection du (des) groupe(s) méthoxy pour préparer un composé de la revendication 1 où X et/ou Y sont —OH.

**Revendications pour l'Etat contractant AT:**

1. Procédé pour la préparation d'un composé de formule:

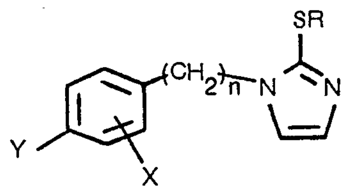

ou X est —H, —OH, halogène, $C_{1-4}$alkyle, —CN, —$NO_2$, —$SO_2NH_2$, $CO_2H$, —$CONH_2$, —CHO, —$CH_2OH$, —$CF_3$, —$OCH_3$, —$SO_2C_{1-4}$alkyle, —$SO_2C_{1-4}$fluoroalkyle, ou —$CO_2C_{1-4}$alkyle ou toute combinaison synthétiquement accessible de celui-ci, jusqu'à substituants

Y est —H, —OH, halogène, $C_{1-4}$alkyle, —CN, —$NO_2$, —$SO_2NH_2$, —$CO_2H$, —$CONH_2$, —CHO, —$CH_2OH$, —$CF_3$, —$SO_2C_{1-4}$alkyle, —$SO_2C_{1-4}$fluoroalkyle, ou —$CO_2C_{1-4}$alkyle; et

R est —H ou $C_{1-4}$alkyle; et

n est 0—4,

ou un hydrate ou, lorsque R est $C_{1-4}$alkyle, un sel d'addition avec un acide pharmaceutiquement acceptable de celui-ci, à condition que, lorsque n est O, Y soit —OH et lorsque n est 1—3, au moins un de Y et X ne soit pas —H, qui comprend,

(a) la réaction d'un composé de formule

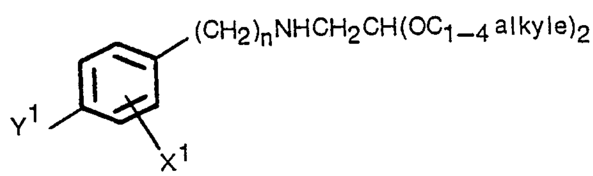

où $Y^1$ est Y mais pas —OH et $X^1$ est X pas pas —OH et n est 0—4, avec un thiocyanate acide; ou,

(b) lorsque n est 0, la cyclisation d'un composé de formule

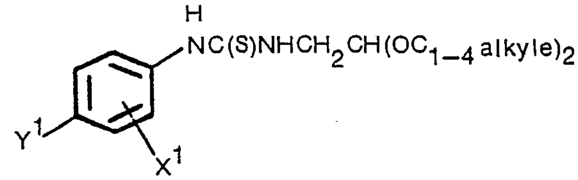

où $Y^1$ est —$OCH_3$ et $X^1$ est tel que défini dans (a) ci-dessus en présence d'un acide et, si on le désire, l'alkylation du composé ainsi formé où R est —H, en un composé où R est $C_{1-4}$alkyle et/ou lorsque $X^1$ et/ou $Y^1$ sont —$OCH_3$, si on le désire, la déprotection du (des) groupe(s) méthoxy pour préparer un composé où X et/ou Y sont —OH.

2. Procéde pour la préparation d'une composition pharmaceutique qui comprend la combinaison d'un composé de structure,

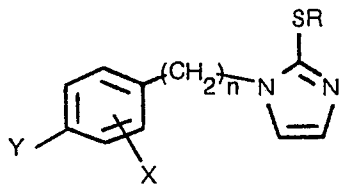

où X, Y, R et n sont tels que définis dans la revendication 1 ou un hydrate ou, lorsque R est $C_{1-4}$alkyle, un sel d'addition avec un acide pharmaceutiquement acceptable de celui-ci à condition que, lorsque n est O, Y soit OH, avec un excipient pharmaceutiquement acceptable dans ce but.